(19) **Europäisches Patentamt** / **European Patent Office** / **Office européen des brevets**

(11) **EP 4 000 531 A1**

(12) **EUROPEAN PATENT APPLICATION**

(43) Date of publication:
**25.05.2022 Bulletin 2022/21**

(21) Application number: **20206848.2**

(22) Date of filing: **11.11.2020**

(51) International Patent Classification (IPC):
**A61B 8/08** *(2006.01)*    **A61B 8/00** *(2006.01)*
**A61B 8/06** *(2006.01)*

(52) Cooperative Patent Classification (CPC):
**A61B 8/4254; A61B 8/0891; A61B 8/483;**
A61B 8/06; A61B 8/488

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **FERNANDO, Shakith Devinda**
**5656 AE Eindhoven (NL)**
• **VAN BREE, Karl Catharina**
**5656 AE Eindhoven (NL)**

(74) Representative: **Philips Intellectual Property & Standards**
**High Tech Campus 52**
**5656 AG Eindhoven (NL)**

(54) **METHODS AND SYSTEMS FOR TRACKING A MOTION OF A PROBE IN AN ULTRASOUND SYSTEM**

(57)    The invention provides for a method of generating a tracked imaging region representing ultrasound data acquired from a subject. The method comprises obtaining ultrasound data acquired from an imaging region by way of an ultrasound probe. A first image and a second image of a surface, acquired during the acquisition of the ultrasound data, are obtained by way of an image sensor coupled to the ultrasound probe. The first image and the second image are compared and a first motion component of the ultrasound probe is computed based on the comparison.

A second motion component of the ultrasound probe, acquired during the acquisition of the ultrasound data, is then obtained by way of an inertial measurement unit coupled to the image sensor.

The first motion component and the second motion component are combined, thereby generating a motion of the ultrasound probe, which is then combined with the ultrasound data from the imaging region, thereby generating a tracked imaging region.

FIG. 2

**Description**

FIELD OF THE INVENTION

The invention relates to the field of ultrasound imaging, and more specifically to the field of probe tracking in ultrasound imaging.

BACKGROUND OF THE INVENTION

Ultrasound imaging is used in many interventional and diagnostic applications. Often, for interventional or diagnostic use-cases, finding and identifying the relevant organs is critical.

For example, one particular use-case may include finding the carotid artery and the right location within the carotid artery for obtaining a blood flow measurement. In a further example, a use-case may include locating the desired region for needle access for applying regional anesthesia. In a further example, a use-case may include the cannulation of tubes into the veins of a subject. However, interpreting ultrasound images in the use-cases described above is challenging and requires a skilled professional to interpret the images accurately.

Ultrasound systems vary from high-end cart based or static machines to portable, low-cost solutions. There is a trend towards developing mobile ultrasound imaging solutions that allow handheld ultrasound devices to be connected to a mobile device, such as a smartphone.

Currently, using ultrasound probes for finding and identifying vascular access is difficult, particularly in low-cost, portable ultrasound systems. The radiologist must mentally build and map the vessel of interest in a 3D mental picture whilst moving (both translating and rotating) the probe in a trial and error manner. This may be with, or without, color Doppler data depending on the application. After finding the vessel, the radiologist must then move the ultrasound probe along the vessel while moving (both translating and rotating) the probes in a trial and error manner to find the desired location, such as a bifurcation point of a vessel. The radiologist then moves the probe until the vessel under investigation is parallel to the steering angle of the probe and manually measure and enter the value for the diameter of the vessel in order to obtain an estimated blood flow measurement.

The method described above is error-prone, time-consuming, unintuitive and requires a trained and experienced professional in order to achieve an accurate result. Even with sufficient training a small error in the angle of the probe may lead to a significant error in the blood flow measurement.

There is therefore a need for a means of accurately tracking the motion of a probe for use in an imaging method.

SUMMARY OF THE INVENTION

The invention is defined by the claims.

According to examples in accordance with an aspect of the invention, there is provided a method for generating a tracked imaging region representing ultrasound data acquired from a subject, the method comprising:

obtaining ultrasound data acquired from an imaging region by way of an ultrasound probe;
obtaining a first image of a surface acquired during the acquisition of the ultrasound data by way of an image sensor coupled to the ultrasound probe;
obtaining a second image of the surface acquired during the acquisition of the ultrasound data by way of the image sensor;
comparing the first image and the second image;
computing a first motion component of the ultrasound probe based on the comparison;
obtaining a second motion component of the ultrasound probe acquired during the acquisition of the ultrasound data by way of an inertial measurement unit coupled to the image sensor;
combining the first motion component and the second motion component, thereby generating a motion of the ultrasound probe; and
combining the ultrasound data from the imaging region and the motion of the ultrasound probe, thereby generating a tracked imaging region.

The method provides for a means of accurately tracking the motion of an ultrasound probe, and the imaging region defined by the field of view of the ultrasound probe, using a combination of a motion signal derived from an image sensor and a motion signal obtained from an inertial measurement unit, thereby increasing the accuracy of the motion tracking of the ultrasound probe.

By aligning the incoming ultrasound data based on the combined motion signals of the ultrasound probe, the region being imaged by the ultrasound probe may be accurately tracked within a 3D coordinate system.

In an embodiment, the method further comprises generating a 3D ultrasound volume based on the tracked imaging region by combining the ultrasound data of the tracked imaging region and motion of the ultrasound probe.

In this way, a 3D ultrasound volume may be generated by arranging the obtained ultrasound data within a 3D coordinate system based on the derived motion of the ultrasound probe.

In an embodiment, the method further comprises:

generating an ultrasound image based on the ultrasound data obtained from a tracked imaging region; and generating, in real-time, a live representation of the tracked imaging region within the 3D ultrasound volume based on a combination of the ultrasound image, the 3D ultrasound volume and the motion of the ultrasound probe.

In this way, a live visualization of the current view of the ultrasound probe, i.e. the tracked imaging region, may be displayed within the context of a 3D ultrasound volume, thereby providing for an intuitive visualization system for guiding the user to image a desired region.

In a further embodiment, the ultrasound data comprises Doppler ultrasound data, and wherein generating the 3D ultrasound volume comprises generating a 3D Doppler vessel map based on the Doppler ultrasound data and the motion of the ultrasound probe.

By segmenting the 3D Doppler vessel map based on the Doppler ultrasound data and the motion of the ultrasound probe, the accuracy of the vessel map may be increased and the position of the ultrasound probe when capturing the Doppler ultrasound data may be accounted for.

In a further embodiment, the method further comprises deriving a blood flow measure from the 3D Doppler vessel map, wherein deriving the blood flow measure comprises one or more of:

calculating an angle between an imaging region of the ultrasound probe and the central axis of a vessel based on the motion of the ultrasound probe and adjusting the Doppler ultrasound data based on the calculated angle; and calculating a cross sectional area of a vessel based on the 3D Doppler vessel map and adjusting the Doppler ultrasound data based on the calculated cross sectional area.

In this way, blood flow information may be derived from the vessel map. For example, the angle of the ultrasound probe when acquiring the ultrasound data may be accounted for in the Doppler ultrasound data, thereby improving the accuracy of any derived blood flow measures. Further, the shape of the vessel may also be accounted for in the correction of Doppler data, thereby increasing the accuracy of the derived blood flow measures.

In an embodiment, the method further comprises:

generating a guidance information for positioning an interventional device based on the 3D ultrasound image; and providing the guidance information to a user.

In this way, a user may receive guidance for positioning an interventional device within or outside the field of view of the 3D ultrasound image.

According to examples in accordance with an aspect of the invention, there is provided a computer program comprising computer program code means which is adapted, when said computer program is run on a computer, to implement the steps of:

obtaining ultrasound data acquired from an imaging regions by way of an ultrasound probe; obtaining a first image of a surface acquired during the acquisition of the ultrasound data by way of an image sensor coupled to the ultrasound probe; obtaining a second image of the surface acquired during the acquisition of the ultrasound data by way of the image sensor; comparing the first image and the second image; computing a first motion component of the ultrasound probe based on the comparison; obtaining a second motion component of the ultrasound probe acquired during the acquisition of the ultrasound data by way of an inertial measurement unit coupled to the image sensor; combining the first motion component and the second motion component, thereby generating a motion of the ultrasound probe; and combining the ultrasound data from the imaging region and the motion of the ultrasound probe, thereby generating a tracked imaging region.

In an embodiment, the ultrasound data comprises Doppler ultrasound data, and wherein the computer program is adapted, when said computer program is run on a computer, to implement the step of generating the 3D ultrasound volume by generating a 3D Doppler vessel map based on the Doppler ultrasound data and the motion of the ultrasound probe, wherein the ultrasound data comprises Doppler ultrasound data, and wherein the computer program is further adapted, when said computer program is run on a computer, to implement the step of generating the 3D ultrasound volume by generating a 3D Doppler vessel map based on the Doppler ultrasound data and the motion of the ultrasound probe.

In an embodiment the computer program is adapted, when said computer program is run on a computer, to further implement the step of deriving a blood flow measure from the 3D Doppler vessel map, wherein deriving the blood flow measure comprises one or more of:

calculating an angle between an imaging region of the ultrasound probe and the central axis of a vessel based on the motion of the ultrasound probe and adjusting the Doppler ultrasound data based on the calculated angle; and calculating a cross sectional area of a vessel based on the 3D Doppler vessel map and adjusting the Doppler ultrasound data based on the calculated cross sectional area.

According to examples in accordance with an aspect of the invention, there is provided a computer-readable storage medium comprising instructions which, when executed by a computer, cause the computer to carry out the steps of:

obtaining ultrasound data acquired from an imaging region by way of an ultrasound probe;
obtaining a first image of a surface acquired during the acquisition of the ultrasound data by way of an image sensor coupled to the ultrasound probe;
obtaining a second image of the surface acquired during the acquisition of the ultrasound data by way of the image sensor;
comparing the first image and the second image;
computing a first motion component of the ultrasound probe based on the comparison;
obtaining a second motion component of the ultrasound probe acquired during the acquisition of the ultrasound data by way of an inertial measurement unit coupled to the image sensor;
combining the first motion component and the second motion component, thereby generating a motion of the ultrasound probe; and
combining the ultrasound data from the imaging region and the motion of the ultrasound probe, thereby generating a tracked imaging region.

In an embodiment, the computer-readable storage medium further comprises instructions which, when executed by a computer, cause the computer to carry of the step of generating a 3D ultrasound volume based on the tracked imaging region by combining the ultrasound data of the tracked imaging region and motion of the ultrasound probe, wherein the ultrasound data comprises Doppler ultrasound data, and wherein the computer-readable storage medium further comprises informations which, when executed by a computer, cause the computer to carry out the step of generating the 3D ultrasound volume by generating a 3D Doppler vessel map based on the Doppler ultrasound data and the motion of the ultrasound probe.

In an embodiment the computer-readable storage medium further comprises instructions which, when executed by a computer, cause the computer to carry out the further step of deriving a blood flow measure from the 3D Doppler vessel map, wherein deriving the blood flow measure comprises one or more of:

calculating an angle between an imaging region of the ultrasound probe and the central axis of a vessel based on the motion of the ultrasound probe and adjusting the Doppler ultrasound data based on the calculated angle; and calculating a cross sectional area of a vessel based on the 3D Doppler vessel map and adjusting the Doppler ultrasound data based on the calculated cross sectional area.

According to examples in accordance with an aspect of the invention, there is provided a processing system for use in an ultrasound system and for generating a tracked imaging region representing from ultrasound data acquired from an imaging regions, the processing system comprising:

an input to receive a first image, a second image, a second motion component and ultrasound data; and
a processor coupled to the input to:

compare the first image and the second image;
compute a first motion component of the ultrasound probe based on the comparison;
combine the first motion component and the second motion component, thereby generating a motion of the ultrasound probe; and
combine the ultrasound data from the imaging region and the motion of the ultrasound probe, thereby generating a tracked imaging region.

According to examples in accordance with an aspect of the invention, there is provided an ultrasound imaging system comprising:

the processing system described above;
an ultrasound probe adapted to acquire ultrasound data;
an image sensor coupled to the ultrasound probe and adapted to acquire images of a surface; and
an inertial measurement unit coupled to the image sensor and adapted to acquire the second motion component.

In an embodiment, the processor is further adapted to generate a 3D ultrasound volume based on the tracked imaging region by combining the ultrasound data of the tracked imaging region and motion of the ultrasound probe.

In an embodiment, the processor is further adapted to:

generate an ultrasound image based on the ultrasound data obtained from a tracked imaging region; and
generate, in real-time, a live representation of the tracked imaging region within the 3D ultrasound volume based on a combination of the ultrasound image, the 3D ultrasound volume and the motion of the ultrasound probe.

In an embodiment, the ultrasound data comprises Doppler ultrasound data, and wherein the processor is adapted to, when generating the 3D ultrasound volume, generate a 3D Doppler vessel map based on the Doppler ultrasound data and the motion of the ultrasound probe, and optionally wherein the system further comprises a user interface adapted to receive a user input, and wherein the processor is adapted to derive a blood flow measure from the 3D Doppler vessel map in response to receiving the user input at the user interface, wherein deriving the blood flow measure comprises one or more of:

calculating an angle between an imaging region of the ultrasound probe and the central axis of a vessel based on the motion of the ultrasound probe and adjusting the Doppler ultrasound data based on the calculated angle; and
calculating a cross sectional area of a vessel based on the 3D Doppler vessel map and adjusting the Doppler ultrasound data based on the calculated cross sectional area.

In an embodiment, the system further comprises a display unit, and wherein the processor is further adapted to instruct the display unit to display one or more of:

the 3D ultrasound volume; and
the 3D Doppler vessel map.

In an embodiment, the image sensor comprises one or more of:

a camera;
a 3D camera; and
a LIDAR sensor.

In an embodiment, the inertial measurement unit comprises one or more of:

an accelerometer; and

a gyroscope.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows an ultrasound diagnostic imaging system to explain the general operation;
Figure 2 shows a schematic representation of an ultrasound imaging system according to an aspect of the invention;
Figure 3 shows a method for determining a position of an ultrasound imaging probe with respect to a surface; and
Figure 4 shows a method for generating a 3D ultrasound image.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides for a method of generating a tracked imaging region representing ultrasound data acquired from a subject. The method comprises obtaining ultrasound data acquired from an imaging region by way of an ultrasound probe. A first image and a second image of a surface, acquired during the acquisition of the ultrasound data, are obtained by way of an image sensor coupled to the ultrasound probe. The first image and the second image are compared and a first motion component of the ultrasound probe is computed based on the comparison.

A second motion component of the ultrasound probe, acquired during the acquisition of the ultrasound data, is then obtained by way of an inertial measurement unit coupled to the image sensor.

The first motion component and the second motion component are combined, thereby generating a motion of the ultrasound probe, which is then combined with the ultrasound data from the imaging region, thereby generating a tracked imaging region.

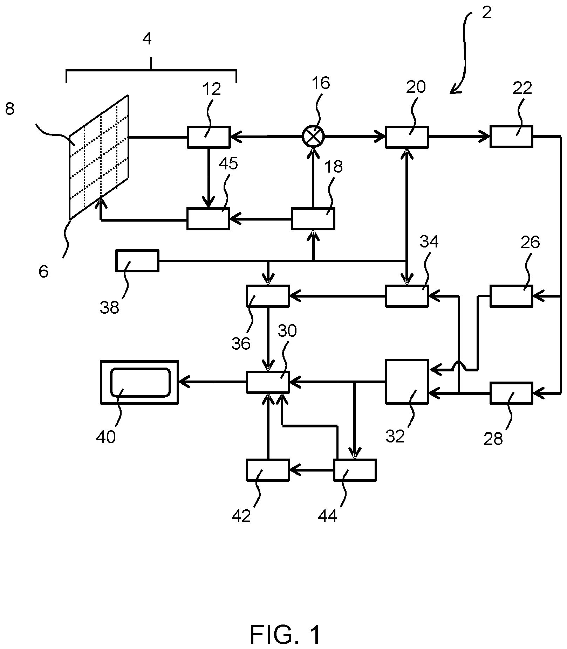

The general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

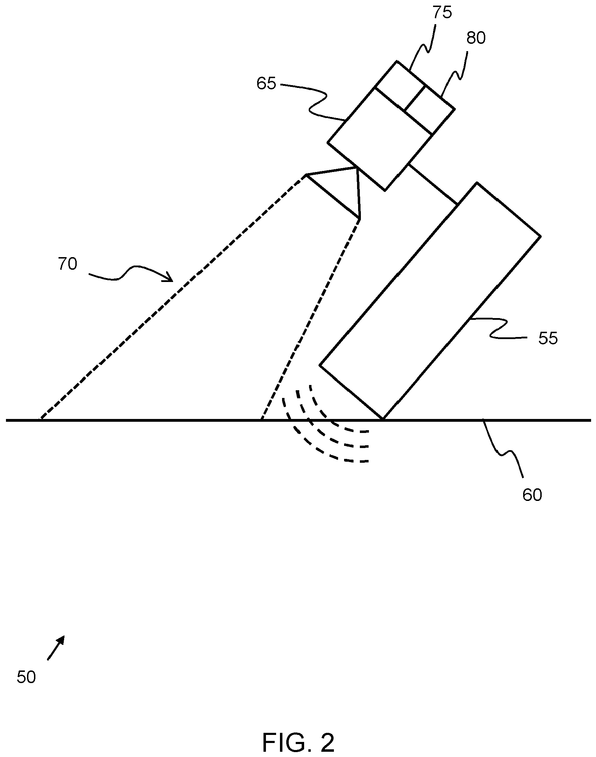

Figure 2 shows a schematic representation of an ultrasound imaging system 50 according to an aspect of the invention.

The system comprises an ultrasound probe 55 adapted to acquire ultrasound data adjacent a surface 60 of a region of interest of a subject undergoing investigation. For example, the surface may be the skin of a subject. The ultrasound probe may be any ultrasound transducer-based system suitable for obtaining ultrasound data from the surface of a subject. For example, the ultrasound probe may have a 1D ultrasound transducer array, a 2D ultrasound transducer array or a 3D matrix ultrasound probe and comprise part of a static or portable ultrasound system. The US system may be one as explained with reference to Figure 1.

The system further includes an image sensor 65 permanently or releasably coupled to the ultrasound probe 55 and adapted to acquire images of the surface 60 within a field of view 70. The image sensor may be any suitable image sensor, such as a visible spectrum camera, a 3D camera, a time of flight camera, a LIDAR camera or an infra-red camera. In addition, the system comprises an inertial measurement unit 75 permanently or releasably coupled to the probe and/or image sensor 65 and adapted to acquire a motion signal of the image sensor. The inertial measurement unit may be any suitable motion sensor, such as an accelerometer or a gyroscope.

In an example, the ultrasound probe may be connected to a smart device, such as a smartphone, which comprises an image sensor and an inertial measurement unit.

The image sensor and inertial measurement unit are suitable for or even adapted to simultaneous localization and mapping (SLAM) of the ultrasound probe. SLAM can be implemented a processor also part of the system such as for example that of the smartphone. Alternatively or additionally such SLAM may be implemented in a processor of the ultrasound probe or a hybrid solution may be employed. In the case of the smartphone the smartphone processor may perform the SLAM processing. Alternatively, dedicated SLAM hardware on the phone may also implement the SLAM processing. In the case that the SLAM functionality is implemented in the probe, dedicated SLAM hardware may be integrated into the probe for the SLAM processing.

During use, the image sensor captures images of the surface, which may be compared to each other to determine the first motion component of the ultrasound probe. This is because the image sensor as well as the inertial unit have a fixed orientation to the ultrasound probe. The first motion component of the ultrasound probe derived from the comparison between the images may include six degrees of freedom, which comprise three translational degrees of freedom and three angular degrees of freedom. Simultaneously, the inertial measurement unit measures the second motion components, which may comprise 3 angular degrees of freedom. The first motion component and the second motion component may be combined immediately upon acquisition, but they may also be combined at a later stage. Since an ultrasound image recorded by the probe has a known orientation with respect to the probe, knowing the location and orientation of the probe also provides information on the location and orientation of the ultrasound image recorded at a particular time stamp. The first and second motion component may thus be coupled to or associated with recorded US images at relevant time stamps.

Accordingly, by combining the first and second motion components in order to derive the motion of the ultrasound probe, the robustness and accuracy of the overall motion signal may be improved. For example, movement artefacts present in the images captured by the image sensor, which in turn may lead to errors in the estimated motion of the ultrasound probe, may be compensated for by including the independent inertial measurement unit 75 coupled to the image sensor.

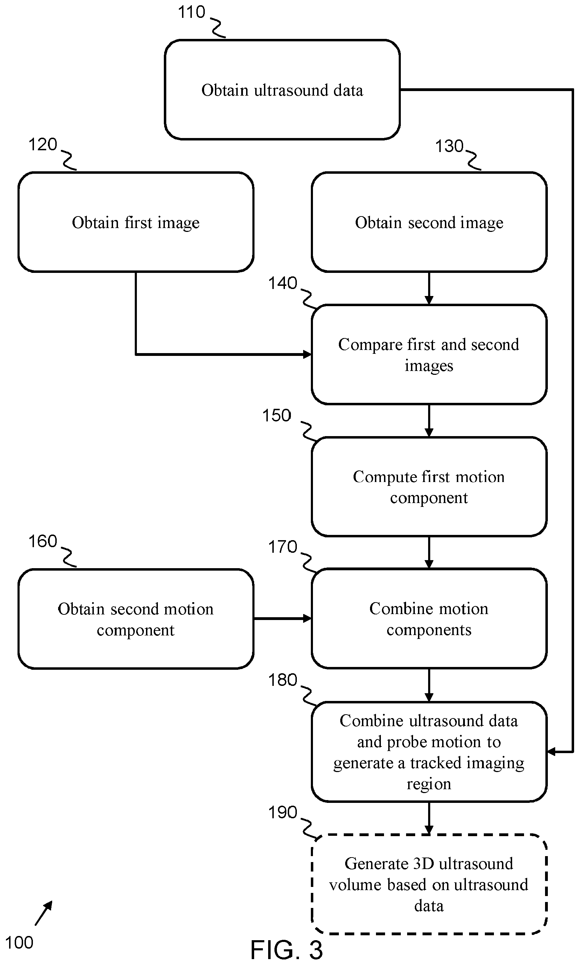

The system 50 further comprises a processor 80 in communication with the image sensor 65 and the inertial measurement unit 75. The operation of the processor is described further below with reference to Figure 3. The processor may optionally be in communication with the ultrasound probe; however, this is not necessary for the methods and operations described herein. Rather, the combination of image sensor and inertial measurement sensor, and in some cases the processor, may only require a physical connection to the ultrasound probe in order to derive the motion of the ultrasound probe, meaning that the components of this system may be incorporated into any existing ultrasound probe or system. It should be noted that, although the processor 80 is shown schematically connected to the system 50 in Figure 2, the processor may alternatively be a remote processor that is in wired or wireless communication with the system 50. A processor that may be used is further described herein below.

The image sensor 65, inertial measurement unit 75 and processor 80 may form part of an integrated unit, such as a smartphone or SLAM unit, that may be mounted to the ultrasound probe in any suitable manner. In the example where the image sensor 65, inertial measurement unit 75 and processor 80 form part of an integrated unit, the integrated unit may comprise any other suitable component for the operation of the integrated unit, such as a battery and/or a Wi-Fi communication unit.

The image sensor 65, inertial measurement unit 75 and processor 80 provide the motion data of the ultrasound probe (for example in the form of a set of coordinates describing the combination of the six degrees of freedom of motion from the images captured by the image sensor and the three degrees of freedom of motion captured by the inertial measurement unit) and the ultrasound probe 55 provides a stream of ultrasound data to form ultrasound images. Both the motion data and the ultrasound data may be provided to a separate visualization unit, for example in an asynchronous manner, which combines the ultrasound data and the motion data. The ultrasound data and motion data may be provided to the visualization unit using any suitable wired or wireless connection, such as a Wi-Fi connection. The ultrasound data and the motion data may be synchronized prior to being combined.

Figure 3 shows a method 200 for generating a tracked imaging region representing ultrasound data acquired from a subject, which may be executed by processor 80 as discussed above.

The method begins in step 110 by obtaining ultrasound data acquired from an imaging region by way of an ultrasound probe. The ultrasound data may comprise any ultrasound data type, such as B-mode ultrasound data, M-mode ultrasound data and Doppler ultrasound data, for example color Doppler ultrasound data. This is of coarse dependent on the type of diagnosis to be performed. Thus anatomical imaging may only require regular intensity data while flow measurements alternatively or additionally may require the recording of Doppler data.

In step 120, a first image of the surface is obtained by way of the image sensor coupled to the ultrasound probe and in step 130, a second image of the surface is obtained by way of the image sensor. The first and second images are acquiring during the acquisition of the ultrasound data. A parameter indicative of relative timing of the recordings may be acquired and associated with one or more of the images.

In step 140, the first image and the second image are compared to each other and in step 150, a first motion component of the ultrasound probe is calculated based on the comparison. For example, corresponding features within the first and second images and the location of said features within each image may form part of the comparison. The difference between the locations between the two images may be derived from the comparison.

Steps 120 to 150 may occur concurrently with step 110. In other words, the first and second images may be obtained, and the first motion component derived, as the ultrasound data is being acquired.

The features may include any identifiable feature within the field of view of the image sensor. For example, in the case that the surface is the skin of a subject, the feature may comprise one or more of: a skin color; a change in skin color; a texture; a natural mark; an artificial mark; a scar; hair; pores and the like. The features may be identified by way of any suitable image processing method. Segmentation may be performed. The identification may be done manually via user input or automatically.

In step 160, a second motion component of the image sensor is obtained by way of an inertial measurement unit coupled to the image sensor and in step 170 the first and second motion components are combined to generate a motion of the ultrasound probe.

Steps 160 and 170 may occur concurrently with steps 110 and 120 to 150. In other words, motion sensor may obtain the second motion component, which may then be combined with the first motion component, as the ultrasound data is being acquired. The time stamps referred to herein before with regard to obtaining the first and second image may thus be those used for the respective inertial measurements and thus second motion components too.

Put another way, as the ultrasound data is acquired, both the image sensor and the inertial movement sensor may acquire the first and second motion components, respectively. The first and second motion components are then combined to obtain the motion of the ultrasound probe, which may then be used to locate and track the ultrasound data obtained by the probe within a 3D coordinate system.

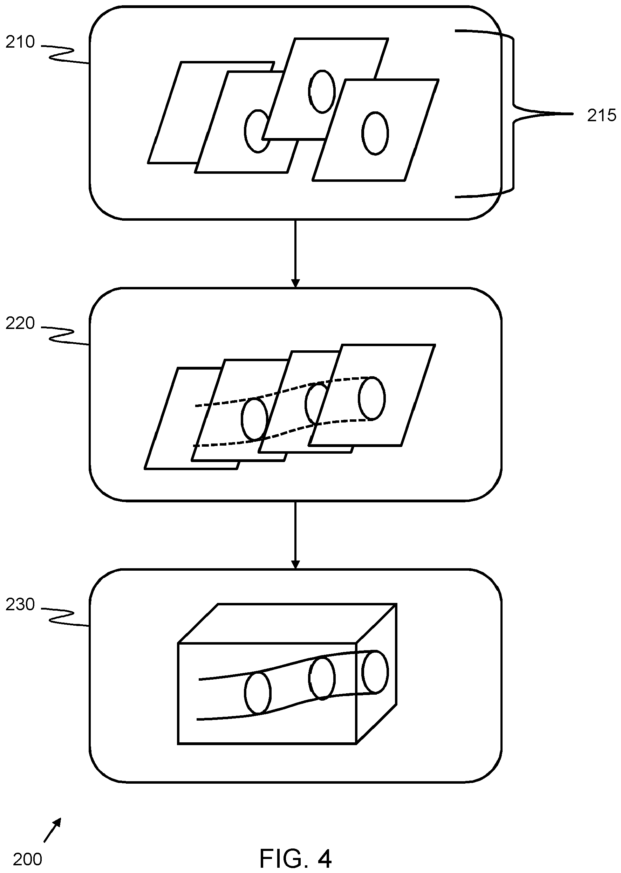

In step 180, the ultrasound data from the imaging region is combined based on the motion data of the ultrasound probe, thereby generating a tracked imaging region. Optionally, in step 190, a 3D ultrasound volume may be generated based on the so combined ultrasound data. These steps are discussed in further detail below with reference to Figure 4.

In a further step which is not shown in the Figure 3, the tracked imaging region or 3D ultrasound volume is output to a user via a suitable output interface such as a display device.

It should be noted that, although shown as separate steps for the purposes of clarity, the steps of obtaining the ultrasound data, obtaining the first and second images and computing the first motion component, obtaining the second motion component and combining the first and second motion components may be performed simultaneously or near-simultaneously.

The method described for example with reference to Figure 3 may further include generating an ultrasound image based on the ultrasound data obtained from the tracked imaging region and generating, in real-time, a live representation of the tracked imaged region within the 3D ultrasound volume based on a combination of the ultrasound image, the 3D ultrasound volume and the motion of the ultrasound probe.

Put another way, the current field of view of the ultrasound probe may be rendered and displayed to the user in the context of the 3D ultrasound volume. The positioning of the current field of view relative to the 3D ultrasound volume may be determined based on the motion of the ultrasound probe derived as described above. The means of visualization described above is intuitive to a user as there is a direct correlation between the way in which the user moves the ultrasound probe and how the current field of view, i.e. the imaging region, moves in the context of the 3D ultrasound volume. Thus, the user may more easily position, or be guided to position, the ultrasound probe in a region of interest.

The method is advantages as it may provide a real time good quality visualization of a tracked US imaging region using a relatively simple US system comprising only one camera and an inertial unit. Thus, advantageously the method and system enable a portable US system with relatively simple components to be used for a volumetric US measurement. An example simple system may be a 2D ultrasound probe equipped with only one camera and inertial unit comprised within for example a portable device such as phone or tablet coupled to the probe.

Figure 4 shows a method 200 for generating a 3D ultrasound image using the motion of the ultrasound probe as obtained in Figure 3.

The method begins in step 210 by obtaining ultrasound data from an imaging region by way of the ultrasound probe. For example as described with reference to Figure 3. Figure 4 shows a schematic representation of a plurality of imaging regions in the form of a plurality of imaging planes 215, which may, for example, have been acquired at different points in time as the ultrasound probe moved across a surface of the subject. As can be seen from the Figure, the imaging planes are not correctly aligned to each other.

In step 220, the ultrasound data is spatially registered based on the combined motion of the ultrasound probe and in step 230, a 3D ultrasound image of a volume is generated based on the spatially registered ultrasound data. The 3D ultrasound image may be generated by way of interpolation between the spatially registered ultrasound data, but this may not be needed perse.

In this way, the quality of the final 3D ultrasound image is not dependent on the user or their level of expertise. Rather, the systems and methods described above, and below, may provide for a means of obtaining a high-quality 3D ultrasound image by a user of any skill level.

The ultrasound data may comprise any ultrasound data type, such as B-mode ultrasound data, M-mode ultrasound data and Doppler ultrasound data, for example color Doppler ultrasound data.

In the case that the ultrasound data comprises Doppler ultrasound data, the 3D ultrasound image may comprise a 3D Doppler vessel map. The 3D Doppler vessel map may be obtained by segmenting the vessel structure from the Doppler ultrasound data which depicts the blood flow within a vessel and the motion of the ultrasound probe. The segmentation may be done by methods known in the art.

For example, each Doppler slice of the vessel may be tracked in a global coordinate system as explained with reference to Figures. 3 and 4, which may then be used to accumulate a 3D vessel map. The vessel map may be part of a tracked US region and the vessel map may be shown in a tracked volume.

In an example, the system may be adapted to locate a vessels' split point such as a bifurcation point of a vessel within the 3D vessel map. For example, contour detection may be applied to the ultrasound data in each imaging plane to identify the vessel perimeter. The image moments, being a weighted average of the pixels of an image based on the intensity of the pixels, may then be used to find the center of the contours of the vessel in an image. The center of each contour in each image slice may be tracked over time to identify a vector, referred to as the center vector within for example the global coordinate system. Temporal filtering may also be applied to the center vector to remove noise, but may not always be necessary.

When the center count, i.e. the number of detected centers (or associated center vectors), changes a vessel split point may be detected. Thus for example when the number of centers switches from 1 to more than 1 (such as 2), or from more than 1 (such as from 2) to 1, in e.g. the filtered center vector, the vessels splitting point (bifurcation point in case of split into two vessels) can be identified. After all, in the split point the vessel with one contour splits into two or more contours or vice versa multiple contours merge into one.

A blood flow measure may be derived from the 3D Doppler vessel map. As the 3D Doppler vessel map is based on the motion corrected ultrasound data, the accuracy of the derived blood flow measure is improved and the number of errors is reduced.

In an example, deriving the blood flow measure comprises calculating an angle between an imaging plane of the ultrasound probe and the central axis (e.g. parallel to the vessel) of a vessel based on the motion of the ultrasound probe and adjusting the Doppler ultrasound data based on the calculated angle. In this way, even if the ultrasound data is collected at an incorrect angle, the Doppler ultrasound data may be corrected before the blood flow measure is derived, thereby accounting for the erroneous acquisition angle.

In a specific example, the blood flow measure may be calculated as follows. The center vector may be computed as described above and the dominant eigenvector representing the center vectors over all of the images is computed. The eigenvector is then treated as the vessel vector, which defines a central axis of the vessel. For example, the eigenvector may be calculated at a particular region of interest necessary for a blood flow determination. The eigenvector calculation may thus be performed using part of the recorded and motion corrected ultrasound data to recover the vessel vector most representative for the region needed for the blood flow calculation.

Given the vessel vector and the vectors representing the ultrasound imaging plane, the scan angle of the ultrasound probe maybe computed. The scan angle, which is user dependent, may be used to correct the Doppler color velocities in the given imaging plane on a per pixel basis. The sum of the corrected velocities of the pixels in the contour may then form the final blood flow measurement.

The correction of the Doppler color velocities may be performed using the following formula:

$$\text{Corrected\_doppler\_values} = \text{original\_doppler\_values} / \text{Cosine\_Angle} ,$$

wherein:

$$\text{Cosine\_Angle} = \text{dot}(\text{Normalized\_Plane\_Vector}, \text{VesselVector}) / \text{Magnitude}(\text{VesselVector})$$

In a further example, the Doppler ultrasound data may be adjusted based on the measured cross-sectional area of a vessel as measured from the 3D Doppler vessel map.

For example, the diameter or radius of the vessel cross-section may be estimated visually, or measured on the ultrasound image. Typically, the measured radius is converted to cross-sectional area, using the assumption that the vessel cross section is an ideal circle. However, in reality, the vessel cross section is often not circular and may be deformed by plaque, organs and the like.

The sum of the adjusted velocities, as derived from the formula above, derived from the pixels in the Doppler ultrasound data may form the final blood flow measurement. The sum of the pixels is computed from a pixel mask that is segmented from the vessel slice, in the ultrasound imaging plane, or the 3D Doppler vessel map. This mask contains a physically accurate vessel cross section, which may account for vessel deformation and lead to a more accurate flow measurement based on the true vessel cross section rather than using an estimated circle.

The 3D Doppler vessel map may also be used to guide a user in an interventional process. For example, a user may be required to insert a needle at a certain point in a vessel. Based on the 3D vessel map, the system may determine the optimal point for insertion and optimal insertion angle, which may then be provided to the user performing the interventional procedure. The guidance information may be provided to the user, for example, by one or more of: a visual means, such as a visual projection onto the surface; an audible means; a tactile means; and the like. The guidance information may be provided to the user in a visual manner by way of a display or by way of an augmented reality headset.

Further, an interventional device may include a position detection system, which may inform the processor of the location of the interventional device, which may then form the basis of the guidance information. Alternatively, the interventional device may be located within the field of view of the ultrasound probe, in which case the location of the interventional device for generating the guidance information may be derived from the ultrasound data.

In a further example, the guidance information may include guidance as to how to move the probe, for example in order to revisit a previously investigated area of the subject vasculature. In other words, the 3D Doppler vessel map may be used as the basis for generating a guidance information so as to guide the user to revisit a previously measured portion of the subject. As the 3D Doppler vessel map is generated based on the motion of the ultrasound probe, the representation of the vessel structure and the associated guidance is accurate.

The methods described above may be performed by any suitable processor of any suitable computer or computing system, such as a smartphone, laptop, personal computer, processing server or cloud-based processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A method (100) for generating a tracked imaging region representing ultrasound data acquired from a subject, the method comprising:

   obtaining (110) ultrasound data acquired from an imaging region by way of an ultrasound probe;
   obtaining (120) a first image of a surface acquired during the acquisition of the ultrasound data by way of an image sensor coupled to the ultrasound probe;
   obtaining (130) a second image of the surface acquired during the acquisition of the ultrasound data by way of the image sensor;
   comparing (140) the first image and the second image;
   computing (150) a first motion component of the ultrasound probe based on the comparison;
   obtaining (160) a second motion component of the ultrasound probe acquired during the acquisition of the ultrasound data by way of an inertial measurement unit coupled to the image sensor;
   combining (170) the first motion component and the second motion component, thereby generating a motion of the ultrasound probe; and
   combining (180) the ultrasound data from the imaging region based on the motion of the ultrasound probe, thereby generating a tracked imaging region.

2. A method as claimed in claim 1, wherein the method further comprises generating (190) a 3D ultrasound volume comprising the tracked imaging region by combining the ultrasound data of the tracked imaging region based on the motion of the ultrasound probe.

3. A method (100) as claimed in claim 1 or 2, wherein the method further comprises:

   generating an ultrasound image based on the ultrasound data obtained from a tracked imaging region; and
   generating, in real-time, a representation of the tracked imaging region within the 3D ultrasound volume based on a combination of the ultrasound image, the 3D ultrasound volume and the motion of the ultrasound probe.

4. A method (100) as claimed in any of claims 2 to 3, wherein the ultrasound data comprises Doppler ultrasound data, and wherein generating the 3D ultrasound volume comprises generating a 3D Doppler vessel map based on the Doppler ultrasound data and the motion of the ultrasound probe.

5. A method (100) as claimed in claim 4, wherein the method further comprises deriving a blood flow measure from the 3D Doppler vessel map, wherein deriving the blood flow measure comprises one or more of:

   calculating an angle between an imaging region of the ultrasound probe and the central axis of a vessel based on the motion of the ultrasound probe and adjusting the Doppler ultrasound data based on the calculated angle; and
   calculating a cross sectional area of a vessel based on the 3D Doppler vessel map and adjusting the Doppler ultrasound data based on the calculated cross sectional area.

6. A method (100) as claimed in any of claims 2 to 5, wherein the method further comprises:

   generating guidance information for positioning an interventional device based on the 3D ultrasound volume; and
   providing the guidance information to a user.

7. A computer program comprising computer program code means which is adapted, when said computer program is run on a computer, to implement the steps of:

   obtaining ultrasound data acquired from an imaging region by way of an ultrasound probe;
   obtaining a first image of a surface acquired during the acquisition of the ultrasound data by way of an image sensor coupled to the ultrasound probe;
   obtaining a second image of the surface acquired during the acquisition of the ultrasound data by way of the image sensor;
   comparing the first image and the second image;
   computing a first motion component of the ultrasound probe based on the comparison;
   obtaining a second motion component of the ultrasound probe acquired during the acquisition of the ultrasound data by way of an inertial measurement unit coupled to the image sensor;

combining the first motion component and the second motion component, thereby generating a motion of the ultrasound probe; and

combining the ultrasound data from the imaging region and the motion of the ultrasound probe, thereby generating a tracked imaging region.

8. A computer program as claimed in claim 7, wherein the computer program is adapted, when said computer program is run on a computer, to implement the step of generating a 3D ultrasound volume based on the tracked imaging region by combining the ultrasound data of the tracked imaging region and motion of the ultrasound probe, wherein the ultrasound data comprises Doppler ultrasound data, and wherein the computer program is further adapted, when said computer program is run on a computer, to implement the step of generating the 3D ultrasound volume by generating a 3D Doppler vessel map based on the Doppler ultrasound data and the motion of the ultrasound probe.

9. A computer program as claimed in claim 8, wherein the computer program is adapted, when said computer program is run on a computer, to further implement the step of deriving a blood flow measure from the 3D Doppler vessel map, wherein deriving the blood flow measure comprises one or more of:

calculating an angle between an imaging region of the ultrasound probe and the central axis of a vessel based on the motion of the ultrasound probe and adjusting the Doppler ultrasound data based on the calculated angle; and

calculating a cross sectional area of a vessel based on the 3D Doppler vessel map and adjusting the Doppler ultrasound data based on the calculated cross sectional area.

10. A computer-readable storage medium comprising instructions which, when executed by a computer, cause the computer to carry out the steps of:

obtaining ultrasound data acquired from an imaging regions by way of an ultrasound probe;

obtaining a first image of a surface acquired during the acquisition of the ultrasound data by way of an image sensor coupled to the ultrasound probe;

obtaining a second image of the surface acquired during the acquisition of the ultrasound data by way of the image sensor;

comparing the first image and the second image;

computing a first motion component of the ultrasound probe based on the comparison;

obtaining a second motion component of the ultrasound probe acquired during the acquisition of the ultrasound data by way of an inertial measurement unit coupled to the image sensor;

combining the first motion component and the second motion component, thereby generating a motion of the ultrasound probe; and

combining the ultrasound data from the imaging region and the motion of the ultrasound probe, thereby generating a tracked imaging region.

11. A computer-readable storage medium as claimed in claim 10, wherein the computer-readable storage medium further comprises instructions which, when executed by a computer, cause the computer to carry of the step of generating a 3D ultrasound volume based on the tracked imaging region by combining the ultrasound data of the tracked imaging region and motion of the ultrasound probe, wherein the ultrasound data comprises Doppler ultrasound data, and wherein the computer-readable storage medium further comprises informations which, when executed by a computer, cause the computer to carry out the step of generating the 3D ultrasound volume by generating a 3D Doppler vessel map based on the Doppler ultrasound data and the motion of the ultrasound probe.

12. A computer-readable storage medium as claimed in claim 11, wherein the computer-readable storage medium further comprises instructions which, when executed by a computer, cause the computer to carry of the further step of deriving a blood flow measure from the 3D Doppler vessel map, wherein deriving the blood flow measure comprises one or more of:

calculating an angle between an imaging plane of the ultrasound probe and the central axis of a vessel based on the motion of the ultrasound probe and adjusting the Doppler ultrasound data based on the calculated angle; and

calculating a cross sectional area of a vessel based on the 3D Doppler vessel map and adjusting the Doppler ultrasound data based on the calculated cross sectional area.

13. A processing system (80) for use in an ultrasound system and for generating a tracked imaging region representing ultrasound data acquired from an imaging region, the processing system comprising:

an input to receive a first image, a second image, a second motion component and ultrasound data; and
a processor coupled to the input to:

compare the first image and the second image;
compute a first motion component of the ultrasound probe based on the comparison;
combine the first motion component and the second motion component, thereby generating a motion of the ultrasound probe; and
combine the ultrasound data from the imaging region and the motion of the ultrasound probe, thereby generating a tracked imaging region.

14. An ultrasound imaging system (50) comprising:

the processing system (80) claimed in claim 13;
an ultrasound probe (55) adapted to acquire ultrasound data;
an image sensor (65) coupled to the ultrasound probe and adapted to acquire images of a surface; and
an inertial measurement unit (75) coupled to the image sensor and adapted to acquire the second motion component.

15. An ultrasound imaging system (50) as claimed in claim 14, wherein the processor is further adapted to generate a 3D ultrasound volume based on the tracked imaging region by combining the ultrasound data of the tracked imaging region and motion of the ultrasound probe.

16. An ultrasound imaging system (50) as claimed in claim 15, wherein the processor is further adapted to:

generate an ultrasound image based on the ultrasound data obtained from a tracked imaging region; and
generate, in real-time, a live representation of the tracked imaging region within the 3D ultrasound volume based on a combination of the ultrasound image, the 3D ultrasound volume and the motion of the ultrasound probe.

17. An ultrasound imaging system (50) as claimed in any of claims 14 to 16, wherein the ultrasound data comprises Doppler ultrasound data, and wherein the processor is adapted to, when generating the 3D ultrasound volume, generate a 3D Doppler vessel map based on the Doppler ultrasound data and the motion of the ultrasound probe, and optionally wherein the system further comprises a user interface adapted to receive a user input, and wherein the processor is adapted to derive a blood flow measure from the 3D Doppler vessel map in response to receiving the user input at the user interface, wherein deriving the blood flow measure comprises one or more of:

calculating an angle between an imaging region of the ultrasound probe and the central axis of a vessel based on the motion of the ultrasound probe and adjusting the Doppler ultrasound data based on the calculated angle; and
calculating a cross sectional area of a vessel based on the 3D Doppler vessel map and adjusting the Doppler ultrasound data based on the calculated cross sectional area.

18. An ultrasound imaging system (50) as claimed in claims 15 and 17, wherein the system further comprises a display unit, and wherein the processor is further adapted to instruct the display unit to display one or more of:

the 3D ultrasound volume; and
the 3D Doppler vessel map.

19. An ultrasound imaging system (50) as claimed in any of claims 14 to 18, wherein the image sensor (65) comprises one or more of:

a camera;
a 3D camera; and
a LIDAR sensor.

20. An ultrasound imaging system (50) as claimed in any of claims 14 to 19, wherein the inertial measurement unit (75) comprises one or more of:

an accelerometer; and
a gyroscope.

FIG. 1

FIG. 2

110

Obtain ultrasound data

120

Obtain first image

130

Obtain second image

140

Compare first and second images

150

Compute first motion component

160

Obtain second motion component

170

Combine motion components

180

Combine ultrasound data and probe motion to generate a tracked imaging region

190

Generate 3D ultrasound volume based on ultrasound data

100

FIG. 3

210

215

220

230

200

FIG. 4

Europäisches
Patentamt

European
Patent Office

Office européen
des brevets

## EUROPEAN SEARCH REPORT

**Application Number**

EP 20 20 6848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/016185 A1 (STOLKA PHILIPP JAKOB [US] ET AL) 17 January 2013 (2013-01-17) | 1-4,6-8, 10,11, 13-16, 18-20 | INV. A61B8/08 A61B8/00 |
| Y A | * paragraphs [0034], [0052], [0063], [0064]; figure 2 * | 4,17 5,9,12 | ADD. A61B8/06 |
| X | US 2016/317122 A1 (DOS SANTOS MENDONCA RICARDO PAULO [US] ET AL) 3 November 2016 (2016-11-03) * paragraphs [0006], [0007], [0030], [0033] * | 1-3,7, 10, 13-16, 18-20 | |
| Y | WO 2020/083660 A1 (KONINKLIJKE PHILIPS NV [NL]) 30 April 2020 (2020-04-30) * the whole document * | 4,17 | |

**TECHNICAL FIELDS SEARCHED (IPC)**

A61B

The present search report has been drawn up for all claims

| Place of search | Date of completion of the search | Examiner |
|---|---|---|
| Munich | 22 April 2021 | Koprinarov, Ivaylo |

EPO FORM 1503 03.82 (P04C01)

## ANNEX TO THE EUROPEAN SEARCH REPORT
## ON EUROPEAN PATENT APPLICATION NO.

EP 20 20 6848

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2021

| Patent document cited in search report | | Publication date | Patent family member(s) | | Publication date |
|---|---|---|---|---|---|
| US 2013016185 | A1 | 17-01-2013 | CA | 2781427 A1 | 26-05-2011 |
| | | | EP | 2501320 A2 | 26-09-2012 |
| | | | JP | 5763666 B2 | 12-08-2015 |
| | | | JP | 2013511355 A | 04-04-2013 |
| | | | US | 2012253200 A1 | 04-10-2012 |
| | | | US | 2013016185 A1 | 17-01-2013 |
| | | | WO | 2011063266 A2 | 26-05-2011 |
| US 2016317122 | A1 | 03-11-2016 | CN | 108601578 A | 28-09-2018 |
| | | | EP | 3288465 A1 | 07-03-2018 |
| | | | US | 2016317122 A1 | 03-11-2016 |
| | | | US | 2016317127 A1 | 03-11-2016 |
| WO 2020083660 | A1 | 30-04-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5997479 A, Savord
- US 6013032 A, Savord
- US 6623432 B, Powers
- US 6283919 B, Roundhill
- US 6458083 B, Jago
- US 6443896 B, Detmer
- US 6530885 B, Entrekin